# EUROPEAN PATENT SPECIFICATION

(11) **EP 1 795 154 B1**
(45) Date of publication and mention of the grant of the patent: **15.10.2008**
(21) Application number: 06024815.0
(22) Date of filing: 30.11.2006
(51) Int. Cl.: A61F 2/16

(54) **Intraocular lens injecting system**
Injektorsystem für Intraokularlinsen
Système d'injecteur de lentille intraoculaire

(30) Priority: 02.12.2005 JP 2005349481
(43) Date of publication of application: 13.06.2007
(73) Proprietor: NIDEK CO., LTD, Gamagori-shi Aichi (JP)
(72) Inventor: Nagasaka, Shinji, Gamagori-shi Aichi (JP); Sunada, Tsutomu, Gamagori-shi Aichi (JP)
(74) Representative: Schmitz, Hans-Werner

(56) References cited:
- EP-A1- 1 502 559
- WO-A-20/05110289
- WO-A2-20/05023154
- US-A- 6 129 733

## Description

### BACKGROUND OF THE INVENTION

### (1) Field of the Invention

The present invention relates to an intraocular lens injecting system that injects an intraocular lens into an eye.

### (2) Related Art

As a type of operation methods for a cataract, a generally used operation is to extract a crystalline lens that has been clouded from an eye and then inject an intraocular lens into the eye as a substitute for the crystalline lens. In injecting the intraocular lens into an eye, an operator first makes an incision on the eye and then crushes and sucks the clouded crystalline lens through the incision using an ultrasonic cataract surgical apparatus. Subsequently, the intraocular lens is folded and injected into a position where the crystalline lens was through the incision and is opened to be arranged.

As an injector used to inject an intraocular lens into an eye, an injector having a mechanism that prevents an intraocular lens from being folded inside in not use, such as on delivery, and allows it to be folded inside in use (injection) is known in the art (see US 2001/0007942A1 (JP-A-2001-104347)). This type of injector is held by (accommodated in) a holder (or received in a casing) on delivery to prevent damage. Therefore, to use the injector, a user has to detach the injector from the holder, thereafter, make a non-foldable intraocular lens foldable.

The document US6129733 discloses apparatus for holding intraocular lenses and for holding intraocular lens injectors which allow for effective transfer of an intraocular lens to an injector in preparation for a surgical lens injection operation, the wings of the injector being removably attachable to the lens holder.

### SUMMARY OF THE INVENTION

It is an object of the present invention to provide an intraocular lens injecting system that is capable of preparing an intraocular lens for use with ease by a simple configuration.

In order to solve the above problem, the present invention is characterized by having the following arrangement.
(1) An intraocular lens injecting system (100) comprising:
   an injector (50) for injecting a foldable intraocular lens (1) in an eye; and
   a holder (30) for holding the injector;
   wherein the injector includes:
      an inserting portion (11) that includes an injecting port (11a) and is inserted through an incision made on the eye; and
      a lens place portion (12) where the intraocular lens is placed and which is formed on an opposite side to the injecting port of the inserting portion, wherein the lens place portion can be closed in a direction in which the intraocular lens is folded and the placed intraocular lens is folded by closing the lens place portion, and
   wherein the holder includes:
      a holding portion (33a, 33b) detachably holding the injector; and
      exterior force applying means (60) for applying exterior force so as to close the lens place portion in response to detachment of the injector from the holding portion.
(2) The system according to (1), wherein the exterior force applying means applies the exterior force to close the lens place portion by contacting the lens place portion or a convex contact portion (19) provided at the lens place portion when the injector is detached from the holding portion.
(3) The system according to (1) or (2), wherein
   the holder includes a protective wall (32) therearound, and
   the exterior force applying means includes a guide wall (60) provided at the upside of a part of the protective wall.
(4) The system according to any one of (1) to (3), wherein
   the lens place portion includes a fixed part (12a) fixed to the inserting portion and a movable part (12b) coupled to the fixed part through a hinge (13), and
   the exterior force applying means applies the exterior force to move the movable part to the side of the fixed part.
(5) The system according to any one of (1) to (4), wherein the holder includes restricting means (80) for restricting a direction in which the injector is detached from the holding portion.

### BRIEF DESCRIPTION OF THE DRAWINGS

Fig. 1 is a schematic view showing an intraocular lens injecting system according to an embodiment of the invention;
Fig. 2 is a schematic view showing an intraocular lens;
Fig. 3 is a schematic view showing a lens cartridge of an injector;
Figs. 4A and 4B are schematic views showing a handpiece of the injector;
Fig. 5 is a schematic view showing a holder that holds the injector;
Figs. 6A and 6B are views showing a state in which the injector is held by the holder;
Figs. 7A and 7B are views for explaining an operation when the injector (the cartridge) is detached from the holder;
Fig. 8 is a view showing an example in which an restrictor for restricting a direction in which the injector (the cartridge) is detached is provided in the holder; and
Figs 9A and 9B are views showing a main part of another embodiment of the present invention.

### DESCRIPTION OF PREFERRED EMBODIMENTS

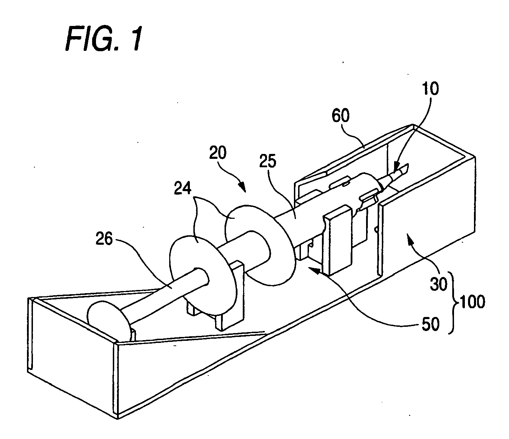

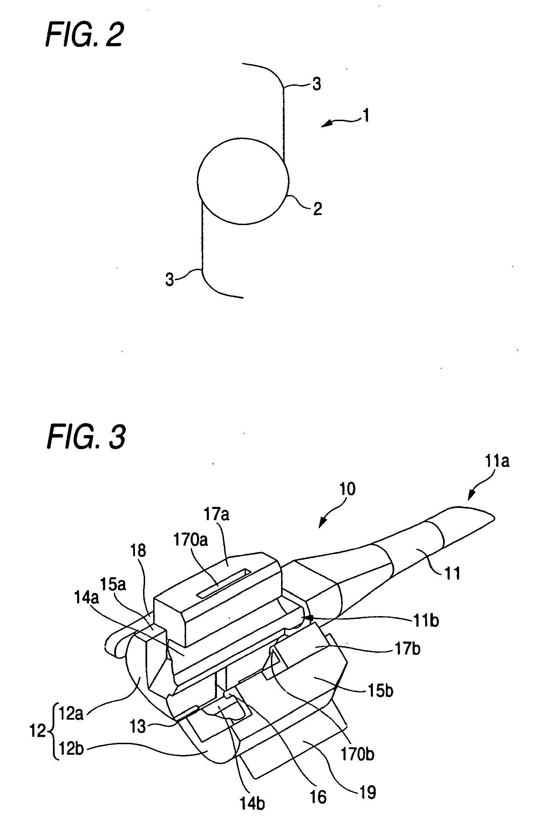

Preferred embodiments of the invention will be described hereafter with reference to accompanying drawings. Fig. 1 is a schematic view showing an intraocular lens injecting system according to an embodiment of the invention. Fig. 2 is a schematic view showing an intraocular lens. An intraocular lens injecting system 100 includes an injector 50 that injects a foldable intraocular lens 1 into an eye, and a holder (a casing) 30 that fixedly holds the injector 50. The injector 50 includes a lens cartridge (a lens holding unit) 10 that holds the intraocular lens 1 and a handpiece (a cartridge holding unit) 20 that holds the cartridge 10.

The intraocular lens 1 includes an optical portion 2 having predetermined refractive power and a supporting portion 3 that supports the optical portion 2 in an eye. The optical portion 2 is formed of an existing material that is used for a foldable optical portion of an intraocular lens, such as, HEMA (Hydroxyethylmethacrylate), a composite of acrylic acid ester and methacrylate, etc. Further, the supporting portion 3 is formed of an existing material that is used for a supporting portion of an intraocular lens, such as PMMA (Polymethylmethacrylate) etc. The shape of the intraocular lens 1 is not limited to those in the embodiments of the invention and any shape may be applied as long as it is foldable.

Fig. 3 is a schematic view of the cartridge 10. Incidentally, upside and downside in the description for Fig. 3 correspond to upside and downside in a normal usage state of the injector 50 (the cartridge 10). The cartridge 10 includes an inserting portion 11 that is to be inserted through an incision on an eye and a lens place portion 12 where the intraocular lens 1 is to be placed (held). The inserting portion 11 is a tube-shaped part tapered at the front end, in which the intraocular lens 1 folded at the lens place portion 12 passes through a hollow portion and is folded smaller, and then extruded out (discharged outside) through an exit (an injecting port) 11a. The lens place portion 12 includes a fixed part 12a that is fixed to the inserting portion 11 and a movable part (an opening/closing part) 12b that is coupled to the fixed part 12a through a hinge 13. The fixed part 12a includes a placing surface 14a inside and the movable part 12b includes a placing surface 14b inside. The intraocular lens 1 is placed on the placing surfaces 14a and 14b in a state that the lens place portion 12 (the fixed part 12a and the movable part 12b) is opened. The intraocular lens 1 placed in the state that the lens place portion 12 is opened is not folded (but, movement in the opening/closing direction of the lens place portion 12 is restricted). When the lens place portion 12 (the fixed part 12a and the movable part 12b) is closed after the intraocular lens 1 is placed, the placing surfaces 14a and 14b form the same shape (half circle) as an inlet 11b of the inserting portion 11 and the intraocular lens 1 placed on the placing surfaces 14a and 14b is folded in the same shape. In other word, the placing surfaces 14a and 14b are curved such that the intraocular lens 1 is not folded when the lens place portion 12 is opened, and it is folded when the lens place portion 12 is closed.

The fixed part 12a is provided with a cover 15a and the movable part 12b is provided with a cover 15b. The covers 15a and 15b cover the upside of the placing surfaces 14a and 14b when the lens place portion 12 is closed. A convex portion 16 extending down toward the placing surfaces 14a and 14b is formed at the side of the cover 15b facing the cover 15a. The convex portion 16 prevents the intraocular lens 1 from being folded in directions, not the appropriate folding direction of the intraocular lens 1, i.e. not the opening/closing direction of the lens place portion 12.

Further, a joint portion 17a extending upward is provided at the side of the cover 15a facing the cover 15b and a joint portion 17b extending upward is provided at the side of the cover 15b facing the cover 15a. The joint portion 17a is provided with a female hook 170a that is engaged (snap-fitted) with a male hook 170b provided to the joint portion 17b. When the lens place portion 12 (the fixed part 12a and the movable part 12b) is closed, the joint portion 17a is jointed with the joint portion 17b and the female hook 170a is engaged with the male hook 170b.

A convex fitting portion 18 is provided at an outside of the fixed part 12a, and a convex contact portion 19 is provided at an outside of the movable part 12b (described hereinafter).

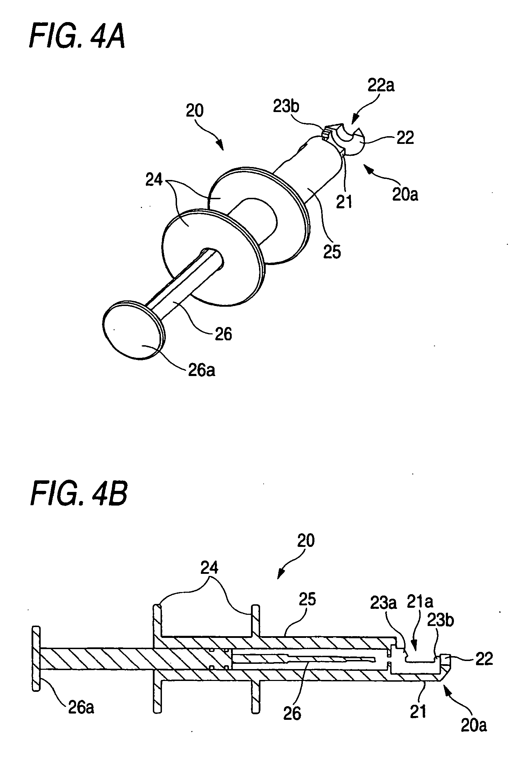

Figs. 4A and 4B are schematic views of the handpiece 20. Fig. 4A is a perspective view and Fig. 4B is a cross-sectional view. The handpiece 20 includes a cylinder (a body unit) 25 where the cartridge 10 is mounted and a piston (a lens extruding unit) 26 that is inserted in the cylinder 25. A mounting portion 20a for the cartridge 10 is formed at the front end of the cylinder 25. Further, a flare-shaped finger portion 24 where a user (an operator) puts his/her fingers to hold the handpiece 20 (the cylinder 25) such as a syringe is formed on the outer periphery of the cylinder 25. A pushing portion 26a for the user to push the piston 26 into the cylinder 25 is provided at the back end of the piston 26.

The mounting portion 20a includes a first receiving portion 21 for the lens place portion 12 (the fixed part 12a) of the cartridge 10 to be placed (fitted) and a second receiving portion 22 for the inserting portion 11 of the cartridge 10 to be placed (fitted). The first receiving portion 21 is shaped like a quadrant circle at the end of the cylinder 25 and the second receiving portion 22 has a letter-C shape in which an arc thereof is a little longer than a half of the circumference around the center axis of the cylinder 25. When the cartridge 10 is mounted on the mounting portion 20a, the bottom of the fixed part 12a contacts with the first receiving portion 21 and the bottom of the inserting portion 11 contacts with the second receiving portion 22. A recess 21a is formed on the first receiving portion 21 by two convex portions 23a and 23b and the fitting portion 18 (see Fig. 3) of the cartridge 10 is fitted in the recess 21a. Further, the inserting portion 11 is fitted in a recess 22a of the second receiving portion 22. As combined as described above, the cartridge 10 is fixedly held at the mounting portion 20a in the state that the lens place portion 12 is opened.

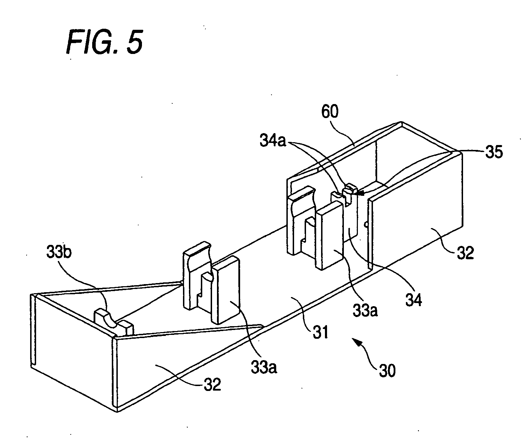

Fig. 5 is a schematic view of the holder 30. A base 31 is provided with a protective wall 32 (a fence) along its edge and includes holding portions 33a and 33b for holding the injector 50. Two holding portions 33a are provided so as to chuck the cylinder 25 and the holding portion 33b supports the piston 26 from underneath. A circular arc spot facing for the cylinder 25 fitted is formed at the upside of the holding portion 33a and another circular arc spot facing for the piston fitted is formed at the upside of the holding portion 33b.

The base 31 is provided with a restrictor 34 that restricts movement of the intraocular lens 1 in a forward/backward direction of the injector 50 (a perpendicular direction to the opening/closing direction of the lens place portion 12) in the lens place portion 12 of the cartridge 10. The restrictor 34 extends in the direction that the injector 50 is detached from the holder 30. A cavity 35 where the intraocular lens 1 (the optical portion 2) is placed is formed on upside of the restrictor 34. The front and back sidewalls 34a of the cavity 35 are curved to fit with the circular optical portion 2 and chuck the optical portion 2 from front and rear when the optical portion 2 is placed in the cavity 35. Further, the bottom of the cavity 35 is more curved than the optical portion 2, so that when the optical portion 2 is placed in the cavity 35, the periphery of the optical portion 2 contacts with the edge inside the cavity 35. Accordingly, the intraocular lens 1 is stably placed (held).

Meanwhile, the width of the restrictor 34 in a left/right direction of the injector 50 (the holder 30) (the opening/closing direction of the lens place portion 12) is smaller than the diameter of the optical portion 2, so that a part of the edge of the optical portion 2 protrudes to the left and right of the cavity 35.

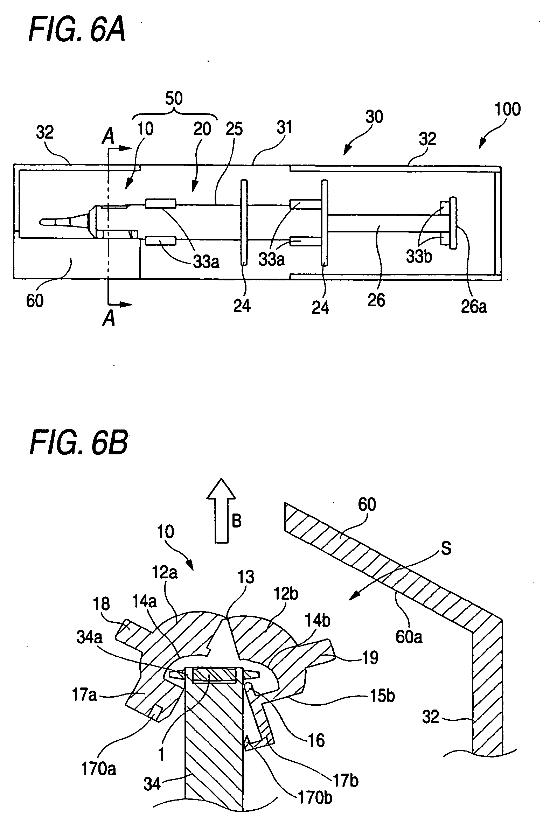

Figs. 6A and 6B are views showing a state in which the injector 50 is held by the holder 30 (i.e. an intraocular lens injecting system 100). Fig. 6A is a plan view taken from above and Fig. 6B is a cross-sectional view taken along a line A-A of Fig. 6A. The lens place portion 12 of the cartridge 10 is opened and the intraocular lens 1 placed in the cavity 35 of the restrictor 34 is covered by the cartridge 10. On the other hand, movement of the piston 26 in the axial direction thereof (the forward/backward direction) is restricted by the holding portion 33b. In the above state, because the front end of the piston 26 is not inserted in the cartridge 10, the intraocular lens 1 is not extruded out by the piston 26.

Fig. 6B is a view showing an arrangement state of the intraocular lens 1 within the injector 50 (the cartridge 10) held by the holder 30. The intraocular lens 1 (the optical portion 2) is placed on the restrictor 34 and covered by the cartridge 10 such that it does not contact with the placing surfaces 14a and 14b in the lens place portion 12. As described above, because the width in the left/right direction of the restrictor 34 is smaller than the diameter of the optical portion 2 and a part of the edge of the optical portion 2 protrudes to the left and right of the restrictor 34, when the injector 50 (the cartridge 10) is detached from the holder 30 (holding portions 33a and 33b) in the direction of an arrow B, the intraocular lens 1 is caught by the placing surfaces 14a and 14b of the cartridge 10 and remains in the lens place portion 12.

A guide wall 60 which is external force applying means for applying external force for making the lens place portion 12 from an opened state to a closed state in response to detachment of the injector 50 (the cartridge 10) from the holder 30 (the holding portions 33a and 33b) is provided at the holder 30. The guide wall 60 is constituted by a part of the protective wall 32 and is provided at the upside of the protective wall 32 at the side of the contact portion 19 of the injector 50 (the cartridge 10) held by (housed in) the holder 30 and is projected inside and inclined upward.

Incidentally, the guide wall 60 have such shape and dimension that the injector 50 (the cartridge 10) does not contact (interfere with) the guide wall 60 when the injector 50 is held by (housed in) the holder 30. The guide wall 60 does not have to be shaped in wall shape. A certain space S is secured when the injector 50 is held by (housed in) the holder 30. Further, the arranging position does not has to be set on the protective wall 32 as long as it is provided at the holder 30.

Further, the restrictor 34 and the guide wall 60 are arranged to keep their positional relationship (interval and the like) so as to allow the movable part 12b to move toward the fixed part 12a when the contact portion 19 contacts the guide wall 60.

The operation of the above described injecting system 100 will be described hereafter.
First, the user takes the injecting system 100 out from a case (not shown) for keeping an aseptic condition, thereafter, detaches the injector 50 (the cartridge 10) from the holder 30 (the holding portions 33a and 33b). In the detaching of the injector 50 (the cartridge 10) as described above, the cartridge 10 is moved in the direction of the arrow B (i.e. upward from the holder 30), and because movement of the intraocular lens 1 placed on the restrictor 34 in the detaching direction is not restricted, the intraocular lens 1 is lifted, contacting with the placing surfaces 14a and 14b. As a result, the intraocular lens 1 is detached from the restrictor 34 (the cavity 35) (i.e. released from the restrictor 34) and remains in the cartridge 10 (the lens place portion 12).

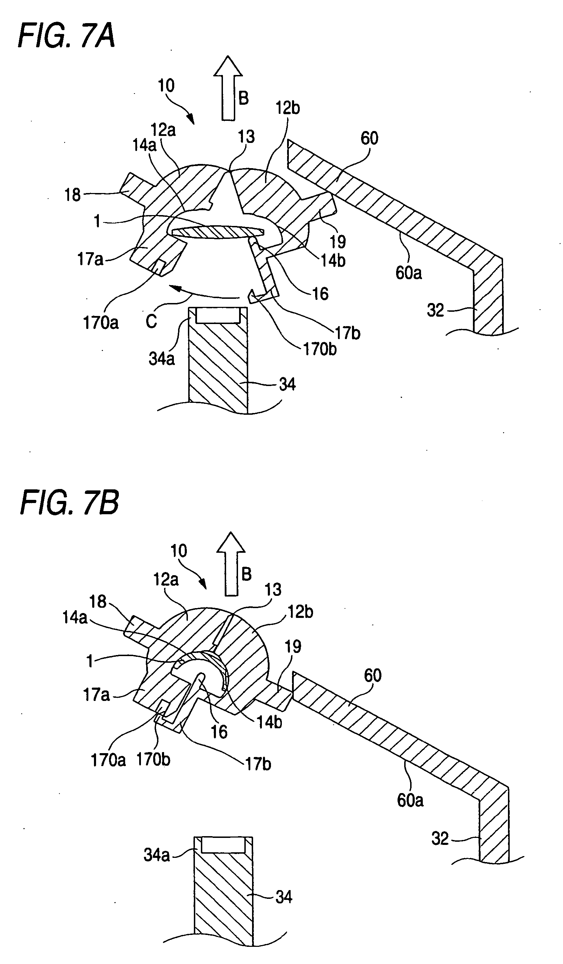

When the injector 50 (the cartridge 10) is moved in the direction of the arrow B, as shown in Fig. 7A, the contact portion 19 contacts an inclined surface 60a of the guide wall 60, and external force applied in a direction in which the movable part 12b approaches the fixed part 12a (the direction of an arrow C) in response to an inclined angle of the inclined surface 60a, thereby the interval between the movable part 12b and the fixed part 12a being narrowed. That is, the guide wall 60 functions so as to guide the lens place portion 12 (the fixed part 12a and the movable part 12b) in a direction to be closed.

When the injector 50 (the cartridge 10) is further moved in the direction of the arrow B from the state of Fig. 7A, as shown in Fig. 7B, the lens place portion 12 (the fixed part 12a and the movable part 12b) is closed and the joint plate portions 17a and 17b are jointed (the male hook 17b is engaged with the female hook 170a). In a state that the lens place portion 12 is closed, the contact portion 9 does not contact the guide wall 60 and the injector 50 (the cartridge) is detached from the holder 30.

In a state that the lens place portion 12 is closed (the fixed part 12a and the movable part 12b are closed), the intraocular lens 1 is pressed by the placing surfaces 14a and 14b and is folded along the placing surfaces 14a and 14b (see Fig. 7B).

As the user pushes the pushing portion 26a to move the piston 26 forward, the piston 26 moves toward the front end in the cylinder 25 and inserts the intraocular lens 1 from the lens place portion 12 into the inserting portion 11. As the intraocular lens 1 is inserted into the inserting portion 11 further, the intraocular lens 1 is folded smaller (wounded) and then the small folded intraocular lens 1 is discharged out of the exit 11a at the front end of the inserting portion 11.

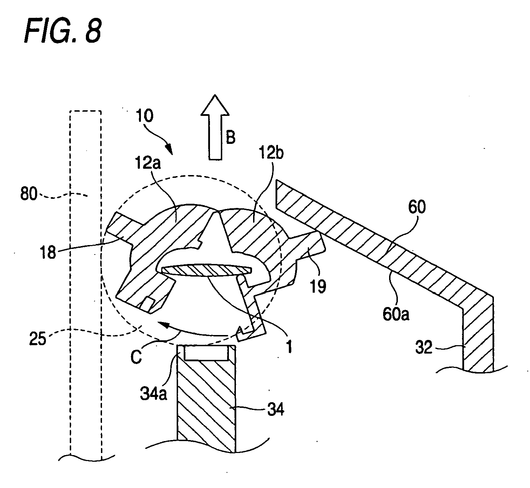

Incidentally, restricting means for restricting a detaching direction of the injector 50 (the cartridge 10) may be provided. For example, as shown in Fig. 8, a restrictor 80 for restricting a movement of the injector 50 in the left/right direction of the cylinder 25 may be provided at the holder 30. With this arrangement, since movement of the injector 50 in a direction other than the direction of the arrow B is restricted when the injector 50 is detached from the holder 30 (the holding portions 33a and 33b), the contact portion 19 surely contacts the guide wall 60.

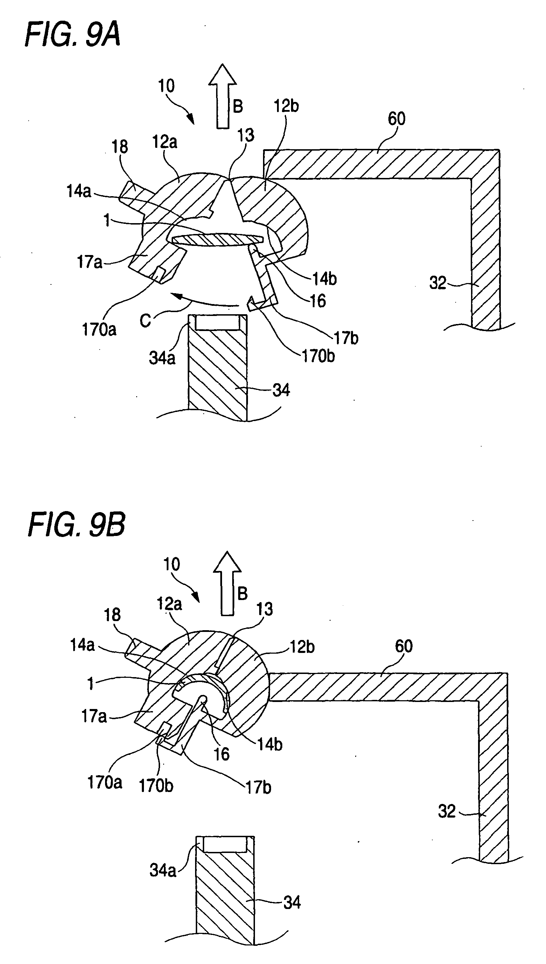

Another embodiment (modification) of the present invention will be briefly described with reference to Figs. 9A and 9B. In this embodiment, the guide wall 60 is provided so as to be perpendicular to the protective wall 32. The contact portion 19 is not provided at the cartridge 10.

When the injector 50 (the cartridge 10) is moved in the direction of the arrow B, as shown in Fig. 9A, the movable part 12b contacts a lower portion of a distal end of the guide wall 60, and external force is applied in the direction in which the movable part 12b approaches the fixed part 12a (the direction of the arrow C), thereby the interval between the movable part 12b and the fixed part 12a being gradually narrowed. When the injector 50 (the cartridge 10) is further moved in the direction of the arrow B from the state of Fig. 9A, as shown in Fig. 9B, the lens place portion 12 (the fixed part 12a and the movable part 12b) is closed and the joint portions 17a and 17b are jointed (the male hook 170b and the female hook 170a are engaged with each other).

Incidentally, in the above description, the intraocular lens 1 is folded by applying external force to the lens place portion 12 in response to the detachment of the injector 50 from the holder 30 (the holding portions 33a and 33b). However, the present invention is not limited thereto, and the intraocular lens 1 may be arranged in a position where the intraocular lens 1 can be pushed out by the piston 26 by applying external force to the lens place portion 12.

As for the embodiments above, the lens cartridge and the handpiece are integrally formed, but not limited thereto, and only the lens cartridge may be held to the holder. According to this configuration, the lens cartridge is held by the holding portions of the holder and movement of the intraocular lens in the lens cartridge is restricted by the restrictor of the holder.

As for the embodiments above, in a state that the injector is held by the holder, the intraocular lens is placed on (holed by) a part (the restrictor 34 in the embodiment) of the holder. However, this is not limited thereto and the intraocular lens may be put in (held by) the lens cartridge (for example, inside the lens place portion 12).

## Claims

1. An intraocular lens injecting system (100) comprising:
an injector (50) for injecting a foldable intraocular lens (1) in an eye; and
a holder (30) for holding the injector;
wherein the injector includes:
an inserting portion (11) that includes an injecting port (11a) and is inserted through an incision made on the eye; and
a lens place portion (12) where the intraocular lens is placed and which is formed on an opposite side to the injecting port of the inserting portion, wherein the lens place portion can be closed in a direction in which the intraocular lens is folded and the placed intraocular lens is folded by closing the lens place portion, and
wherein the holder includes:
a holding portion (33a, 33b) detachably holding the injector; and
guide wall (60) for applying exterior force so as to close the lens place portion by contacting the lens place portion or a convex contact portion (19) provided at the lens place portion at the time of detachment of the injector from the holder, wherein the guide wall have such shape and dimension that the injector does not interfere with the guide wall when the injector is held by the holder and a certain space (S) is secured when the injector is held by the holder.

2. The system according to claim 1, wherein the holder includes a protective wall (32) there around, and
the guide wall (60) is provided at the upside of a part of the protective wall.

3. The system according to any one of claims 1 or 2, wherein
the lens place portion includes a fixed part (12a) fixed to the inserting portion and a movable part (12b) coupled to the fixed part through a hinge (13), and
the guide wall applies the exterior force to move the movable part to the side of the fixed part.

4. The system according to any one of claims 1 to 3, wherein the holder includes restricting means (80) for restricting a direction in which the injector is detached from the holder.

## Patentansprüche

1. Injektorsystem für Intraokularlinsen (100):
- mit einem Injektor (50) zum Injizieren einer faltbaren Intraokularlinse (1) in ein Auge; und
- mit einer Halterung (30) zum Halten des Injektors ;
wobei der Injektor aufweist:
- einen Einsatzbereich (11), der eine Injizierungsöffnung (11a) umfasst und durch einen am Auge erfolgten Schnitt eingeführt wird; und
- einen Linsen-Platzierungsbereich (12), wo die Intraokularlinse platziert wird und der auf einer der Injizierungsöffnung des Einsatzbereichs gegenüberliegenden Seite ausgebildet ist, wobei der Linsen-Platzierungsbereich in einer Richtung geschlossen werden kann, in der die Intraokularlinse gefaltet wird und die platzierte Intraokularlinse durch Schließen des Linsen-Platzierungsbereichs gefaltet wird, und
wobei die Halterung umfasst:
einen Haltebereich (33a, 33b), der den Injektor abnehmbar hält; und
eine Führungswand (60) zum Aufbringen einer äußeren Kraft, um den Linsen-Platzierungsbereich durch eine Kontaktierung des Linsen-Platzierungsbereichs oder eines am Linsen-Platzierungsbereich vorgesehenen konvexen Kontaktbereichs (19) zum Zeitpunkt der Trennung des Injektors von der Halterung zu schließen, wobei die Führungswand eine derartige Form und Abmessung aufweist, dass der Injektor die Führungswand nicht beeinträchtigt, wenn der Injektor durch die Halterung gehalten wird, und ein bestimmter Abstand (S) sichergestellt ist, wenn der Injektor durch die Halterung gehalten wird.

2. System nach Anspruch 1, wobei die Halterung eine Schutzwand (32) darum umfasst, und
die Führungswand (60) auf der Oberseite eines Bereichs der Schutzwand vorgesehen ist.

3. System nach Anspruch 1 oder 2, wobei
der Linsen-Platzierungsbereich einen am Einsatzbereich fixierten feststehenden Bereich (12a) und einen beweglichen Bereich (12b) umfasst, der mit dem feststehenden Bereich über ein Gelenksstück (13) verbunden ist, und
die Führungswand die äußere Kraft aufbringt, um den beweglichen Bereich zur Seite des feststehenden Bereichs zu bewegen.

4. System nach einem der Ansprüche 1 bis 3, wobei die Halterung eine Begrenzungseinrichtung (80) aufweist, um eine Richtung zu begrenzen, in der der Injektor von der Halterung abgenommen wird.

## Revendications

1. Système d'injection de lentille intraoculaire (100) comprenant :
un injecteur (50) pour injecter une lentille intraoculaire pliable (1) dans un oeil ; et
un support (30) pour supporter l'injecteur ;
dans lequel l'injecteur comprend :
une partie d'insertion (11) qui comprend un orifice d'injection (11a) et qui est insérée à travers une incision réalisée sur l'oeil ; et
une partie de placement de lentille (12) où la lentille intraoculaire est placée et qui est formée sur un côté opposé à l'orifice d'injection de la partie d'insertion, dans lequel la partie de placement de lentille peut être fermée dans une direction dans laquelle la lentille intraoculaire est pliée et la lentille intraoculaire placée est pliée en fermant la partie de placement de lentille, et
dans lequel le support comprend :
une partie de support (33a, 33b) supportant l'injecteur de manière détachable; et
une paroi de guidage (60) pour appliquer une force extérieure de manière à fermer la partie de placement de lentille en venant en contact avec la partie de placement de lentille ou une partie de contact convexe (19) prévue au niveau de la partie de placement de lentille à l'instant de détachement de l'injecteur du support, dans lequel la paroi de guidage a une forme et une dimension telles que l'injecteur n'interfère pas avec la paroi de guidage lorsque l'injecteur est supporté par le support et un certain espace (S) est garanti lorsque l'injecteur est supporté par le support.

2. Système selon la revendication 1, dans lequel le support comprend une paroi de protection (32) autour de celui-ci, et
la paroi de guidage (60) est prévue au niveau du côté haut d'une partie de la paroi de protection.

3. Système selon l'une quelconque des revendications 1 et 2, dans lequel
la partie de placement de lentille comprend une partie fixe (12a) fixée à la partie d'insertion et une partie mobile (12b) couplée à la partie fixe par l'intermédiaire d'une articulation (13), et
la paroi de guidage applique la force extérieure pour déplacer la partie mobile vers le côté de la partie fixe.

4. Système selon l'une quelconque des revendications 1 à 3, dans lequel le support comprend des moyens de limitation (80) pour limiter une direction dans laquelle l'injecteur est détaché du support.
